(19) Europäisches Patentamt
European Patent Office
Office européen des brevets

(11) Publication number: **0 129 359**
**A1**

(12) # EUROPEAN PATENT APPLICATION

(21) Application number: 84303725.0

(22) Date of filing: 04.06.84

(51) Int. Cl.³: C 07 D 317/46
C 07 D 307/86, C 07 C 161/00
C 07 D 405/12, C 07 D 413/12
A 01 N 47/24

(30) Priority: 21.06.83 GB 8316845

(43) Date of publication of application:
27.12.84 Bulletin 84/52

(84) Designated Contracting States:
AT BE CH DE FR GB IT LI LU NL SE

(71) Applicant: FBC LIMITED
Hauxton
Cambridge CB2 5HU(GB)

(72) Inventor: Saunders, David Edward
21a Grafton Street
Cambridge CB1 1DS(GB)

(74) Representative: Waldman, Ralph David et al,
Industrial Property Department FBC Limited Chesterford
Park Research Station
Saffron Walden, Essex CB10 1XL(GB)

(54) **Carbamate insecticides.**

(57) Compounds of formula I.

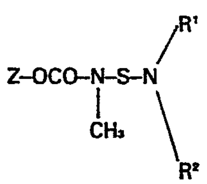

in which a) Z is 2,2-dimethyl-1, 3-benzodioxol-4-yl and $R^1$ and $R^2$ which may be the same or different, are alkyl or cycloalkyl or together with the nitrogen to which they are attached form a 5 to 7 membered ring which may contain other hetero atoms and may be substituted, or

b) Z is the residue of a carbamate insecticide of formula Z-OCONHCH$_3$, $R^1$ is hydrogen and $R^2$ is optionally substituted alkyl or optionally substituted cycloalkyl, have insecticidal and acaricidal activity.

EP 0 129 359 A1

0129359

Case No. 83/16845

CARBAMATE INSECTICIDES

This invention relates to new compounds having insecticidal activity.

The N-methylcarbamates represent a widely used group of insecticides. Examples of commercially available compounds are carbofuran, methomyl, bendiocarb, aldicarb and propoxur. The high mammalian toxicity of many carbamates can restrict their use, particularly against animal ectoparasites. One attempt to overcome this problem is to form sulphenyl derivatives of the carbamates which it is believed are more easily metabolised to the active parent carbamate by insects or acarids than by mammals, thus creating a greater safety margin in use than with the corresponding methylcarbamate withoutthe sulphenyl group. Often however the sulphenyl derivatives will have an insect toxicity which is too low to make the product of practical value against particular insects or other pests, although there are now sulphenyl derivatives of carbofuran which are commercially available.

There is a wide literature on aminosulphenyl derivatives of various carbamates and especially carbofuran. Thus for example the di-n-butylaminosulphenyl derivative which is now marketed and has the common name carbosulfan, is described and claimed in GB 1439112. An improved process for producing carbosulfan is described in

EP 51273. This patent also lists a wide range of carbamates that can be sulphenylated and an even wider range of sulphenylating agents. We have found that bendiocarb can be sulphenylated by a small group of sulphenylating agents to produce compounds having particularly valuable insecticidal activity. There is no suggestion or reason in EP 51273 for selecting this particular combination or that having done so the compounds would have good insecticidal activity. There have been very few specific disclosures of aminosulphenyl derivatives of bendiocarb. Thus EP 7398 claims a double molecule of bendiocarb in which the bendiocarb groups are linked by a sulphur atom. The only other specific disclosures we are aware of, are to arylsulphonylaminosulphenyl derivatives of bendiocarb, as for instance in GB Patent Nos 1403170 and 1454740.

The compounds of the present invention can be prepared in conventional manner by reacting bendiocarb with a sulphenyl halide. We have also found however that the products can be prepared by reacting an imido-sulphenyl derivative of bendiocarb with an amine. We have discovered that this reaction enables the preparation of aminosulphenyl derivatives in which the amine group is monosubstituted. Further we have found that monosubstituted aminosulphenyl derivatives of other

carbamates can be prepared by an analogous reaction. In spite of the wide literature on aminosulphenyl derivatives of carbamates we are not aware of any disclosure of monosubstituted aminosulphenyl derivatives.

Accordingly, the present invention provides a compound of formula I.

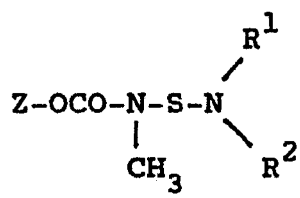

I

in which a) Z is 2,2-dimethyl-1,3-benzodioxol-4-yl and $R^1$ and $R^2$ which may be the same or different, are alkyl or cycloalkyl or together with the nitrogen to which they are attached form a 5 to 7 membered ring which may contain other hetero atoms and may be substituted, or

b) Z is the residue of a carbamate insecticide of formula Z-$OCONCH_3$, $R^1$ is hydrogen and $R^2$ is optionally substituted alkyl or optionally substituted cycloalkyl.

When $R^1$ or $R^2$ are alkyl these are usually of 1 to 8 carbon atoms, expecially 3 to 4 carbon atoms and when substituted, the substitutent is usually alkoxy e.g. methoxy or phenyl. When $R^1$ and $R^2$ form a ring this is preferably morpholino or piperidino. Other rings can

include pyrrolidino. Substitutents can include alkyl or oxo groups and two substituents may form a benzo ring fused to the heterocyclic ring. Cycloalkyl groups are usually of 5 to 7, especially 5 to 6, carbon atoms and may be substituted, e.g. by alkyl, such as methyl.

Z can be the residue of any known carbamate insecticide and is generally selected from phenyl substituted by one or more groups selected from alkyl alkoxy, alkylthio and N,N-dimethylformamido; 1-naphthyl; 2,2-dimethyl-1,3-benzodioxol-4-yl; 2,3-dihydro-2,2-dimethylbenzofuran-7-yl; or an imino group of formula $R^8R^9C=N-$, in which $R^8$ is hydrogen, alkyl or dialkylcarbamoyl and $R^9$ is alkylthio, alkylthioalkyl or alkylsulphonylalkyl.

Preferred groups include those where Z is a)

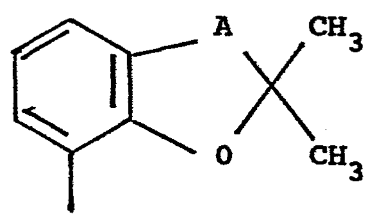

where A is oxygen or methylene;

b) 2-isopropoxyphenyl, or

c) 1-methylthioethylimino.

The compounds of the invention have insecticidal and acaricidal activity and are particularly useful in

combating a variety of economically important insects, and acarids including animal ectoparasites, e.g. Lepidoptera, including Spodoptera littoralis, Heliothis armigera, and Pieris brassicae; Diptera, including Musca domestica, Ceratitis capitata, Erioischia brassicae, Lucilia sericata and Aedes aegypti; Homoptera, including aphids such as Megoura viciae and Nilaparvata lugens; Coleoptera, including Phaedon cochleariae, Anthonomus grandis and corn rootworms (Diabrotica spp. eg. Diabrotica undecimpunctata); Orthoptera, including Blattella germanica; ticks, e.g. Boophilus microplus and lice, including Damalinia bovis and Linognathus vituli.

The invention also includes a pesticidal composition which comprises a compound of formula I in association with an agronomically acceptable diluent or carrier. More than one compound of the invention can, of course, be included in the composition.

In addition the composition can comprise one or more additional pesticides for example compounds known to possess herbicidal, fungicidal, insecticidal, acaricidal or nematicidal properties. Alternatively the compounds of the invention can be used in sequence with the other pesticides. Insecticides and acaricides which can be used in conjunction with the compounds of the invention include natural and synthetic pyrethroids (such as the natural

pyrethrins, allethrin, bioallethrin, resmethrin, bioresmethrin, permethrin, cypermethrin, deltamethrin, fenvalerate, fenpyrithrin, tralocythrin, tralomethrin, flucythrinate, fluvalinate, fenpropathrin, flumethrin, cyfluthrin and cyhalothrin), organophosphorus compounds (such as tetrachlorvinphos, fenitrothion, malathion, dialifos, chlorfenvinphos, demeton-S-methyl phosalone,dichlorovos, bromophos-ethyl, diazinon, dimethoate, sulprofos, acephate, chlorpyrifos, crufomate, heptenophos, naled, phenthoate, phorate, terbufos pirimiphos-ethyl, pirimiphos-methyl, parathion-methyl, temephos, famphur, chlormephos, coumaphos, fenthion and phosmet), carbamates (such as bendiocarb, carbofuran, aldicarb, carbaryl, pirimicarb, promecarb, propoxur, formetanate, methomyl, oxamyl, thiofanox, bufencarb carbosulfan, thiodicarb and dimetilan), chlorinated compounds, (such as toxaphene, endosulphan, HCH and DDT) and miscellaneous compounds including amitraz, chlormethiuron, endosulphan, cyhexatin, diflubenzuron, chlordimeform, benzoximate, dicofol, propargite and clofentezine.

The diluent or carrier in the composition of the invention can be a solid or a liquid optionally in association with a surface-active agent, for example a dispersing agent, emulsifying agent or wetting agent.

Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl-aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block copolymers of ethylene oxide and propylene

oxide, acetylenic glycols such as 2,4,7,9-tetramethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such a amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of insecticidal compounds, for example, a solution, a dispersion, an aqueous emulsion, a dusting powder, a seed dressing, a fumigant, a smoke, a dispersible powder, an emulsifiable concentrate, granules or baits. Moreover it can be in a suitable form for direct application or as a concentrate or primary composition which requires dilution with a suitable quantity of water or other diluent before application.

As a dispersion, the composition comprises a compound of the invention dispersed in a liquid medium, preferably water. It is often convenient to supply the consumer with a primary composition which can be diluted with water to form a dispersion having the desired concentration. The primary composition can be provided in any one of the

following forms. It can be a dispersible solution which comprises a compound of the invention dissolved in a water-miscible solvent with the addition of a dispersing agent. A further alternative comprises a compound of the invention in the form of a finely ground powder in association with a dispersing agent and intimately mixed with water to give a paste or cream which can if desired be added to an emulsion of oil in water to give a dispersion of active ingredient in an aqueous oil emulsion.

An emulsifiable concentrate comprises a compound of the invention dissolved in a water-immiscible solvent together with an emulsifying agent and which is formed into an emulsion on mixing with water. A dusting powder comprises a compound of the invention intimately mixed with a solid pulverulent diluent, for example, kaolin.

A granular solid comprises a compound of the invention associated with similar diluents to those which may be employed in dusting powders, but the mixture is granulated by known methods. Alternatively it comprises the active ingredient adsorbed or absorbed on a pre-granular diluent, for example, Fuller's earth, attapulgite or limestone grit.

A wettable powder usually comprises the active ingredient in admixture with a suitable surfactant and an inert powder diluent such as china clay.

Another suitable concentrate, particularly when the

product is a solid, is a flowable suspension concentrate which is formed by grinding the compound with water, a wetting agent and a suspending agent.

Baits can include an attractant and may comprise a protein hydrolysate e.g. for the control of fruit flies, sugar e.g. for the control of adult Musca spp. or corn cob e.g. for the control of cockroaches.

The concentration of the active ingredient in the composition of the present invention is preferably within the range of 1 to 30 per cent by weight, especially 5 to 30 per cent by weight. In a primary composition the amount of active ingredient can vary widely and can be, for example, from 5 to 95 per cent by weight of the composition.

The compounds of the invention may be prepared by

a) when, $R^1$ is not hydrogen,
by reacting a compound of formula II

$ZOCONHCH_3$ II

with a compound of formula III

$R^1R^2NSQ$ III

where Q is a leaving group such as halogen e.g. chlorine, or

b) by reacting a compound of formula IV

ZOCON-S-Y IV
|
$CH_3$

where Y is a leaving group such as an imido group, with a compound, $R^1R^2NH$. Examples of suitable imido groups include succinimido or phthalimido.

The reaction between compounds II and III is preferably carried out according to the method claimed in European Patent Specification No 51273 in which as catalyst there is used a complex of sulphur dioxide and a trialkylamine. The sulphur dioxide is preferably generated _in situ_ by reacting a compound $(R^1R^2NS)-_2$ with sulphuryl chloride. The disulphide itself can be prepared by reacting $R^1R^2NH$ with sulphur chloride.

Compounds of formula IV may be prepared by reacting a compound of formula II with a compound Y-S-X where X is a leaving group e.g. halogen, especially chlorine.

The invention is illustrated in the following examples. Structures of isolated novel compounds were confirmed by elemental and/or other appropriate analyses.

Example 1

Sulphur monochloride (3.4g) in hexane (8ml) was added dropwise over ½ an hour to a stirred mixture of piperidine (3.7g), hexane (6 ml), sodium hydroxide (2.2g) and water (16 ml) whilst maintaining the temperature at below 40°C with occasional cooling. The mixture was stirred for a further ½ hour at room temperature. The organic layer was

then separated, dried with magnesium sulphate, filtered, placed in a reaction vessel and whilst being stirred, sulphuryl chloride (3g) was added over 20 minutes with the temperature being maintained at 20-25°C. An equal volume of hexane was then added followed by bendiocarb (9g). Triethylamine (7g) was added dropwise to the stirred mixture over ½ hour whilst maintaining the temperature at 20-25°C. The mixture was then stirred at 30-35°C for 2½ hours, cooled to 10°C, water (15ml) added slowly and filtered. The organic layer was separated, dried over magnesium sulphate, filtered and evaporated _in vacuo_. The residual oil was triturated with petroleum ether (bp 40-60°C) to give 2,2-dimethyl-1,3-benzodioxol-4-yl methyl(1-piperidinylthio)carbamate, m.p. 71-2°C. (Compound 1)

Example 2

Triethylamine (5.5g) was added dropwise to a stirred solution of bendiocarb (11.2g) and succinimide -N-sulphenyl chloride (8.3g) in dichloromethane (100 ml) which was maintained at a temperature of 0-10°C. The mixture was then stirred for 2½ hours at room temperature, water was added and the organic layer separated, dried and evaporated _in vacuo_. The residue was triturated with ether and the solid filtered off to give 2,2-dimethyl-1,3-benzodioxol-4-yl methyl(succinimidothio)

carbamate, mp 146-8°C. To a stirred solution of this (2.0g) in dichloromethane (20 ml) was added 2-methoxyethylamine (0.48g) and stirring continued for an hour. The mixture was evaporated in vacuo and the residue extracted with hexane. The extract was evaporated in vacuo to give 2,2-dimethyl-1,3-benzodioxol-4-yl (2-methoxyethylaminothio)methylcarbamate, obtained as an oil. (Compound 2)

Example 3

A mixture of bendiocarb (5g) and triethylamine (3.1ml) in dichloromethane (75ml) was added dropwise with stirring to a solution of phthalimide-N-sulphenyl chloride (4.8g) in dichloromethane (100ml) maintained at a temperature of between -10°C and 0°C. The mixture was stirred at 0°C for 15 minutes and cyclohexylamine (6.5g) then added dropwise. The mixture was stirred at room temperature for one hour, filtered, and evaporated in vacuo. The residue was triturated with hexane, filtered, evaporated in vacuo to give a oil which was purified by medium pressure column chromatography to give 2,2-dimethyl-1,3-benzodioxol-4-yl (cyclohexylaminothio)methylcarbamate, m.p. 84-5°C (Compound 3)

Example 4

In a similar manner starting from the appropriate N-methyl carbamate insecticide and using one of the procedures outlined in the preceding examples the following products were obtained.

In the table under the heading Z:-

A = 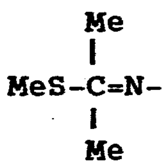

G = 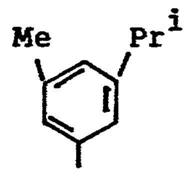

B = 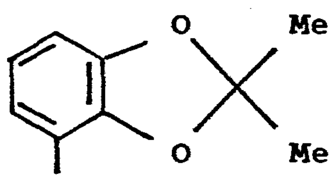

M = 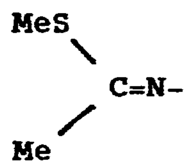

C = 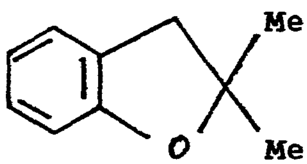

O = 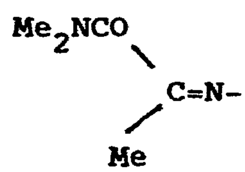

D = 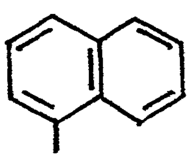

P = 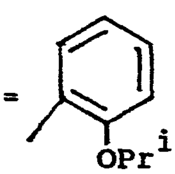

F = 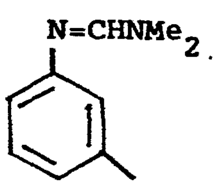

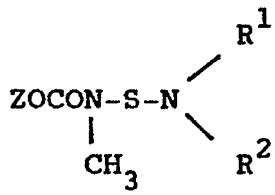

| Compound No | $R^1$ | $R^2$ | Z | mp(°C) |
|---|---|---|---|---|
| 4 | $Bu^n$ | $Bu^n$ | B | oil |
| 5 | Et | Et | B | oil |
| 6 | $-(CH_2)_4-$ | | B | oil |
| 7 | $Pr^n$ | $Pr^n$ | B | oil |
| 8 | $n$-$C_5H_{11}$ | $n$-$C_5H_{11}$ | B | oil |
| 9 | H | $Bu^t$ | B | oil |
| 10 | H | $Bu^n$ | B | 39-40 |
| 11 | Me | $Bu^n$ | B | oil |
| 12 | $Bu^n$ | Et | B | oil |
| 13 | Me | Me | B | oil |
| 14 | $-CH(CH_3)(CH_2)_4-$ | | B | oil |

| Compound No | $R^1$ | $R^2$ | Z | mp(°C) |
|---|---|---|---|---|
| ·15 | 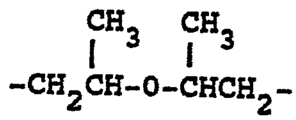 | | B | oil |
| 16 | $-CH_2CH_2OCH_2CH_2-$ | | B | 52-3 |
| 17 | H | $Pr^i$ | B | 53 |
| 18 | $Pr^i$ | $Pr^i$ | B | 53-4 |
| 19 | H | cyclohexyl | D | 75-7 |
| 20 | H | cycloheptyl | B | 84-6 |
| 21 | H | cyclohexyl | O | 125-6 |
| 22 | H | benzyl | B | oil |
| 23 | Me | cyclohexyl | B | oil |
| 24 | H | 2-methyl-cyclohexyl | B | oil |
| 25 | H | 3,3,5-tri-methyl-cyclohexyl | B | 91-2 |
| 26 | H | -CH(Me)Ph | B | oil |
| 27 | H | $Bu^s$ | B | oil |
| 28 | H | $n-C_8H_{17}$ | B | oil |
| 29 | H | cyclopentyl | B | oil |

| Compound No | $R^1$ | $R^2$ | Z | mp(°C) |
|---|---|---|---|---|
| 30 | H | $Bu^i$ | B | 79-82 |
| 31 | $-CHCH(Me)O(CH_2)_2-$ | | B | oil |
| 32 | H | $Pr^i$ | D | oil |
| 33 | Me | $Pr^i$ | B | oil |
| 34 | H | cyclohexyl | M | oil |
| 35 | H | $Pr^i$ | M | 76-7 |
| 36 | H | cyclohexyl | C | 83-4 |
| 37 | H | $Pr^i$ | C | oil |
| 38 | H | $Bu^t$ | C | 76-7 |
| 39 | H | $Bu^t$ | M | 102-3 |
| 40 | H | $Bu^s$ | C | oil |
| 41 | H | 2-methyl-cyclohexyl | C | oil |
| 42 | H | $Bu^s$ | M | 60-2 |
| 43 | H | 2-methyl-cyclohexyl | M | 60-2 |
| 44 | H | cyclopentyl | C | 63-4 |
| 45 | H | $Bu^i$ | C | oil |
| 46 | H | $Pr^i$ | A | 46-7 |

| Compound No | $R^1$ | $R^2$ | Z | mp(°C) |
|---|---|---|---|---|
| 47 | H | $Pr^i$ | P | oil |
| 48 | H | cyclohexyl | P | oil |
| 49 | H | $Bu^n$ | C | oil |
| 50 | 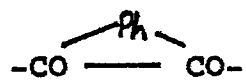 | | B | 144-6 |
| 51 | $-CO(CH_2)_5-$ | | B | glass |
| 52 | $-CH(Et)(CH_2)_4-$ | | B | oil |
| 53 | H | cyclopentyl | F | oil |
| 54 | H | $Bu^s$ | F | oil |
| 55 | H | cyclopentyl | G | oil |
| 56 | H | $Bu^s$ | G | oil |

Example 4

Activity tests

(1) Diabrotica undecimpunctata and (2) Pieris brassicae

Aqueous solvent dispersions containing various concentrations of test compound together with 500 mg of ethylene oxide-nonyl phenol wetting agent per litre were applied in a Potter tower (for a) or by immersion (for b) to:-

(a) 50-100 corn rootworm eggs (Diabrotica undecimpunctata)

in a petri dish lined with damp filter paper; and (b) pieces of cabbage leaf (Brassica spp) each infested with a batch of 25-50 eggs of the cabbage white butterfly (Pieris brassicae). The treated eggs were then (A) held at 22°C for up to 7 days on moist filter paper (for a)or (B) placed on untreated cabbage leaves in petri dishes and held at 22°C for up to 5 days (for b)

The percentage mortality of the eggs was then recorded. Less than 5% mortality resulted in the controls whereas compounds 1-12, 14-19, 23, 26-31, 33, 36, 38-41, and 44-46 had an $LD_{50}$ of less than 1000 mg/litre against (1) and Compounds 1-18 had an $LD_{50}$ of less than 1000 mg/litre against (2).

(3) Musca domestica and (4) Blattella germanica

Aliquots of acetone solutions of test compounds at various concentrations were applied to:-
(a) 9 cm diameter filter papers placed in the bottom of 9 cm diameter glass dishes closed by glass lids, or
(b) glass plates (10 cm x 10 cm). After evaporation of solvent the treated surfaces, together with controls treated with acetone alone, were then infested respectively with:-
(A) adult houseflies, (Musca domestica and held at 22°C for 24 hours (for a); or

(B) second instar nymphs of the German cockroach, (Blattella germanica), retained on the treated surface within PTFE-coated glass rings 6 cm in diameter and held for 24 hours at 22°C (for b).

The percentage mortality of the insects was then recorded. Less than 5% mortality resulted in the control treatments whereas the $LD_{50}$ of compounds 1, 2, 5-18, 21-23, 25, 27-31, 33-40, 42, 44-48 and 50-52 was less than 1000 $mg/m^2$ of filter paper against (3); and the $LD_{50}$ of compounds 1-18, 22-25, 27-48 and 51-52 was less than 500 $mg/m^2$ of glass against (4).

(5) Megoura viciae, (6) Plutella xylostella and (7) Phaedon cochleariae

Aqueous solvent dispersions containing various concentrations of test compound together with 500 mg of the ethylene oxide-nonyl phenol wetting agent per litre were applied to run-off to:-

a) young bean plants, Vicia fabae, which were then infested with wingless bean aphids, Megoura viciae, sprayed beforehand in a Potter tower with 2 ml of the same test formulation;

b) young cabbage plants, Brassica spp., infested with 3rd instar larvae of diamond-backed moth, Plutella xylostella; or

c) young cabbage plants, <u>Brassica spp</u>., which were then infested with 4th instar larvae of the mustard beetle, <u>Phaedon cochleariae</u>, sprayed beforehand in a Potter tower with 2 ml of the same test formulation.

The treated plants, together with controls treated with 500 mg per litre aqueous wetting agent alone, were:-

A) held at 20°C for 24 hours under cylindrical plastic cages closed at the top with gauze (for a);

C) divided up, each leaf being placed separately in a petri dish lined with filter paper and held at 22°C for 3 days (for b and c).

The percentage mortality of the pests was then recorded. Less than 5% mortality resulted in the controls, whereas compounds 1-31, 33-47 and 50-52 had an $LD_{50}$ of less than 1000 mg/litre against (5);

(a); Compounds 1-18, 20-31, 33, 34 and 52 had $LD_{50}$ of less than 1000 mg/litre against (6);

(b); and Compounds 1-18, 20 and 21 had an $LD_{50}$ of less than 1000 mg/litre against (7).

8) <u>Tick tests</u>

a) 9 cm diameter filter papers were impregnated with 1 ml aliquots of acetone solutions of test compound at various concentrations. The papers were allowed to dry and then folded into envelopes in which cattle tick larvae,

(Boophilus microplus) were enclosed and held at 25°C and 80% R.H. for 48 hours. The percentage mortality of tick larvae was then recorded and compared with controls. The controls gave less than 5% mortality whereas compounds 1-52, had an $LD_{50}$ of less than 1000 mg/litre.

b) Groups of 5 mature female cattle ticks were dipped for 10 minutes in aqueous solvent dipersions of test compound at various concentration containing 10 ml acetone and 50 mg of Atlox wetting agent/litre, dried and then placed in individually compartmented plastic containers held at 25°C and >80% R.H., until mortality of ticks or fecundity and viability of eggs produced by survivors could be assessed. The percentage reduction in total reproductive capacity (i.e. the combined effects of adult mortality, reduced fecundity and mortality of eggs) was then recorded and compared with controls. The controls gave less than 5% reduction of reproductive capacity whereas compounds 1, 3-0, 11-14, 16-19, 22, 23, 30, 31, 34, 36-38, 40-42, 44 and 52 gave at least 50% reductions of reproductive capacity at a concentration of 500 mg/litre or less.

Example 5

This example illustrate typical formulations using the compounds of the invention.

| Emulsifiable Concentrates | | % w/v |
|---|---|---|
| Compound 1 | | 25 |
| Agrilan BM[1] | | 10 |
| Xylene | to | 100 |
| | | |
| Compound 1 | | 12½ |
| Ethylan KEO[2] | | 20 |
| Aromatic hydrocarbons | to | 100 |

| Granules | % w/w |
|---|---|
| Compound 1 | 3 |
| Calcium sulphate dihydrate | 97 |

1 Mixture calcium dodecylbenzenesulphonate and nonylphenolethoxylate.

2 Nonylphenolethoxylate.

Similar formulations can be made with the other exemplified compounds.

CLAIMS

1 A compound of formula I.

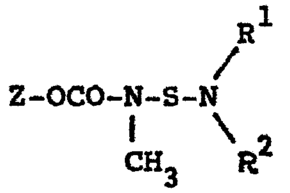

I

in which a) Z is 2,2-dimethyl-1,3-benzodioxol-4-yl and $R^1$ and $R^2$ which may be the same or different, are alkyl or cycloalkyl or together with the nitrogen to which they are attached form a 5 to 7 membered ring which may contain other hetero atoms and may be substituted, or

b) Z is the residue of a carbamate insecticide of formula Z-$OCONHCH_3$, $R^1$ is hydrogen and $R^2$ is optionally substituted alkyl or optionally substituted cycloalkyl.

2) A compound according to claim 1 in which Z is selected from phenyl, substituted by one or more groups selected from alkyl alkoxy, alkylthio and N,N-dimethylformamido; 1-naphthyl; 2,2-dimethyl-1,3-benzodioxol-4-yl; 2,3-dihydro-2,2-dimethylbenzofuran-7-yl; or an imino group of formula $R^8R^9C=N-$, in which $R^8$ is hydrogen, alkyl or dialkylcarbamoyl and $R^9$ is alkylthio, alkylthioalkyl or alkylsulphonylalkyl.

3) A compound according to claim 1 in which $R^1$ and $R^2$ from a morpholino or piperidino ring.

4) A compound accordingly to claim 1 or 2 in which $R^1$ is hydrogen and $R^2$ is $C_{3-4}$ alkyl or $C_{5-6}$ cycloalkyl.

5) A compound according to claim 1, 2 or 4 which Z is

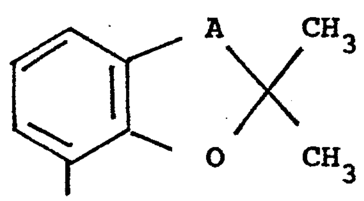

where A is oxygen or methylene.

6) A compound according to claim 1, 2 or 4 in which Z is 2-isopropoxyphenyl

7) A compound according to claim 1, 2 or 4 in which Z is 1-methylthioethylimino.

8) A pesticidal composition which comprises a compound as claimed in claim 1 in association with an agronomically acceptable diluent or carrier.

European Patent Office

# EUROPEAN SEARCH REPORT

0129359
Application number

EP 84 30 3725

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | JOURNAL OF AGRIC. FOOD CHEM., vol. 26, no. 2, 1978, pages 391-395, American Chemical Society, US; E.G. GEMRICH II et al.: "Insecticidal aminothio derivatives of the pesticidal carbamate methomyl" * page 392, table I; pages 393-394 * | 1-3,7, 8 | C 07 D 317/46<br>C 07 D 307/86<br>C 07 C 161/00<br>C 07 D 405/12<br>C 07 D 413/12<br>A 01 N 47/24 |
| X | FR-A-2 337 128 (UPJOHN) * pages 1-3, 19-35, 43-46 * | 1-3,5-8 | |
| X | US-A-4 108 991 (T.R. FUKUTO et al.) * Columns 1,3,4,10 * | 1-3,8 | |
| X | EP-A-0 003 301 (BAYER) * claims * | 1,2,8 | **TECHNICAL FIELDS SEARCHED (Int. Cl. 3)**<br>C 07 D 317/00<br>A 01 N 47/00<br>C 07 D 307/00<br>C 07 C 161/00 |
| D,X | GB-A-1 439 112 (REGENTS OF THE UNIV. OF CALIFORNIA) * pages 1,2,4-7 * | 1-3,8 | |
| D,X | EP-A-0 051 273 (FMC) * pages 10-27 * | 1-3,5-7 | |
| | --- -/- | | |

The present search report has been drawn up for all claims

| Place of search | Date of completion of the search | Examiner |
|---|---|---|
| THE HAGUE | 07-09-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0129359
Application number

EP 84 30 3725



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | DE-A-2 628 574 (UNION CARBIDE) * pages 1-4, 12; page 15, example 10; pages 30, 31-38, claims * --- | 1-4,7,8 | |
| A | EP-A-0 007 398 (HOFFMANN-LA ROCHE) * Pages 1,10,11 * ----- | 1,8 | |

TECHNICAL FIELDS SEARCHED (Int. Cl. 3)

The present search report has been drawn up for all claims

| Place of search | Date of completion of the search | Examiner |
|---|---|---|
| THE HAGUE | 07-09-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document